# EUROPEAN PATENT APPLICATION

(11) **EP 3 978 139 A1**
(43) Date of publication of application: **06.04.2022**
(21) Application number: 20814092.1
(22) Date of filing: 22.05.2020
(51) Int. Cl.: B05B 5/025, B05B 5/053

(54) **ELECTROSTATIC SPRAY DEVICE**

(30) Priority: 31.05.2019 JP 2019103328; 21.05.2020 JP 2020089186
(71) Applicant: Kao Corporation, Chuo-ku Tokyo 103-8210 (JP)
(72) Inventor: OSAWA Kiyoteru, Tokyo 131-8501 (JP)
(74) Representative: Hoffmann Eitle
(86) International application number: PCT/JP2020/020275
(87) International publication number: WO 2020/241483

(57) **Abstract**

An electrostatic spray device, comprising: an electrostatic spray main body capable of containing a liquid containing portion of a cartridge, the cartridge including the liquid containing portion that contains a liquid and a spray unit that sprays the liquid, the electrostatic spray device spraying the liquid by the electrostatic spray main body applying a voltage to the liquid, in which the cartridge has conductivity in at least one portion of the liquid containing portion, and the electrostatic spray main body includes a high-voltage generating unit which generates a high voltage, and a contact portion which is in contact with the portion having conductivity of the liquid containing portion contained in the electrostatic spray main body and applies the high voltage to the liquid in the liquid containing portion through the portion having conductivity.

## Description

### Field of the Invention

The present invention relates to an electrostatic spray device.

### Background of the Invention

In the related art, an electrostatic spray device spraying a liquid by an electrostatic force is known. For example, in JP 2007-521941 A, an electrostatic spray device including a motor, a high-voltage generator, a field electrode, a battery, and the like inside a housing which is made with a dimension that a user is capable of holding with a hand is described. In such an electrostatic spray device, a liquid composition is electrostatically charged by the field electrode to which a high voltage is supplied from the high-voltage generator, and the electrostatically charged liquid composition is sprayed toward a target from a nozzle.

### Summary of the Invention

The present invention relates to an electrostatic spray device, comprising: an electrostatic spray main body configured to be capable of containing a liquid containing portion of a cartridge, the cartridge including the liquid containing portion which contains a liquid and has conductivity in at least one portion and a spray unit which sprays the liquid, the electrostatic spray device spraying the liquid by the electrostatic spray main body applying a voltage to the liquid, the electrostatic spray main body including a high-voltage generating unit which generates a high voltage, and a contact portion which is in contact with the portion having conductivity of the liquid containing portion contained in the electrostatic spray main body and applies the high voltage to the liquid in the liquid containing portion through the portion having conductivity.

In addition, the present invention relates to an electrostatic spray device, comprising: a cartridge including a liquid containing portion which contains a liquid and a spray unit which sprays the liquid; and an electrostatic spray main body capable of containing the liquid containing portion of the cartridge, the electrostatic spray device spraying the liquid by the electrostatic spray main body applying a voltage to the liquid, the liquid containing portion of the cartridge having conductivity in at least one portion, and the electrostatic spray main body including a high-voltage generating unit which generates a high voltage, and a contact portion which is in contact with the portion having conductivity of the liquid containing portion contained in the electrostatic spray main body and applies the high voltage to the liquid in the liquid containing portion through the portion having conductivity.

### Brief Description of the Drawings

Fig. 1A is a perspective view illustrating an electrostatic spray device according to one embodiment of the present invention.
Fig. 1B is a perspective view illustrating a state in which a cap of the electrostatic spray device according to one embodiment of the present invention is removed.
Fig. 2 is an exploded perspective view illustrating an electrostatic spray main body according to one embodiment of the present invention.
Fig. 3A is a configuration diagram illustrating one member of a mounting body of a spray unit of the electrostatic spray device according to one embodiment of the present invention by exploding the mounting body.
Fig. 3B is a configuration diagram illustrating the other member of the mounting body of the spray unit of the electrostatic spray device according to one embodiment of the present invention by exploding the mounting body.
Fig. 4 is a block configuration diagram illustrating a configuration in a housing of the electrostatic spray device according to one embodiment of the present invention.

### Detailed Description of the Invention

In an electrostatic spray device in JP 2007-521941 A, a field electrode is disposed to cover the entire container in which a liquid composition is contained, and is configured to apply approximately a common potential to the liquid composition. For this reason, in the electrostatic spray device in JP 2007-521941 A, there is a problem that the field electrode is required to have a large size, and thus, the dimension (the volume) of a housing increases.

Therefore, the present invention relates to an electrostatic spray device that can be downsized.

That is, the present invention relates to an electrostatic spray device, including: an electrostatic spray main body configured to be capable of containing a liquid containing portion of a cartridge, the cartridge including the liquid containing portion which contains a liquid and has conductivity in at least one portion and a spray unit which sprays the liquid, the electrostatic spray device spraying the liquid by the electrostatic spray main body applying a voltage to the liquid, the electrostatic spray main body including a high-voltage generating unit which generates a high voltage, and a contact portion which is in contact with the portion having conductivity of the liquid containing portion contained in the electrostatic spray main body and applies the high voltage to the liquid containing portion.

In addition, the present invention relates to an electrostatic spray device, including: a cartridge including a liquid containing portion which contains a liquid and a spray unit which sprays the liquid; and an electrostatic spray main body capable of containing the liquid containing portion of the cartridge, the electrostatic spray device spraying the liquid by the electrostatic spray main body applying a voltage to the liquid, the liquid containing portion of the cartridge having conductivity in at least one portion, and the electrostatic spray main body including a high-voltage generating unit which generates a high voltage, and a contact portion which is in contact with the portion having conductivity of the liquid containing portion contained in the electrostatic spray main body and applies the high voltage to the liquid containing portion.

According to an electrostatic spray device according to the present invention, the device can be downsized.

Hereinafter, a preferred embodiment for carrying out the present invention will be described by using the drawings. Note that, the following embodiment does not limit the present invention according to each of the claims, and all combinations of the characteristics described in the embodiment are not essential for means for solving the problems of the present invention.

### [Overall Configuration and Operation Outline of Electrostatic Spray Device]

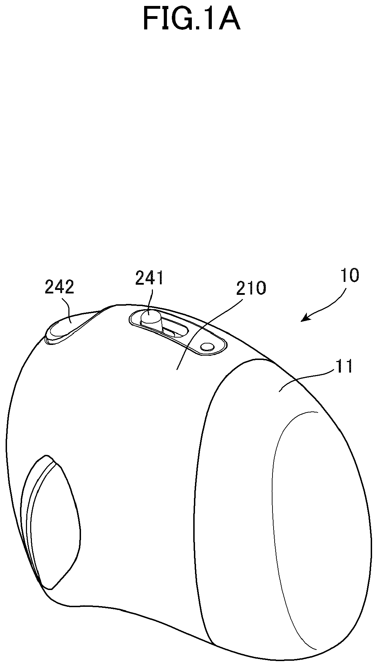

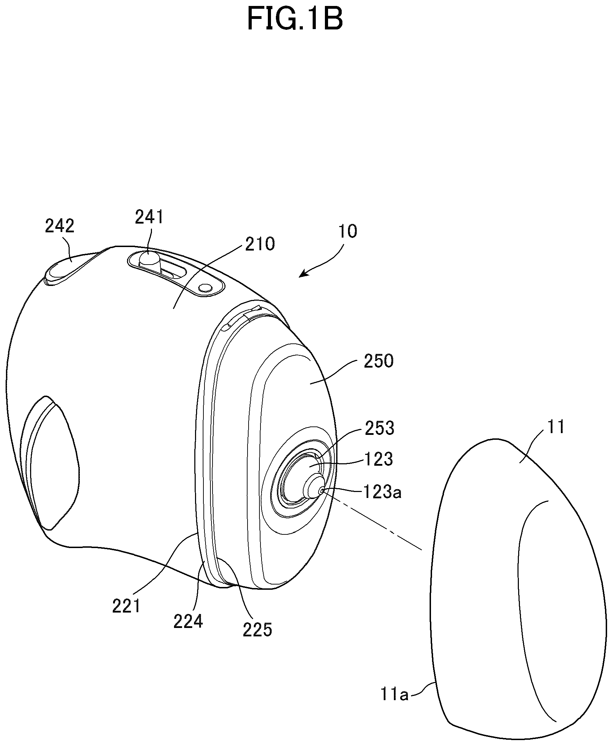

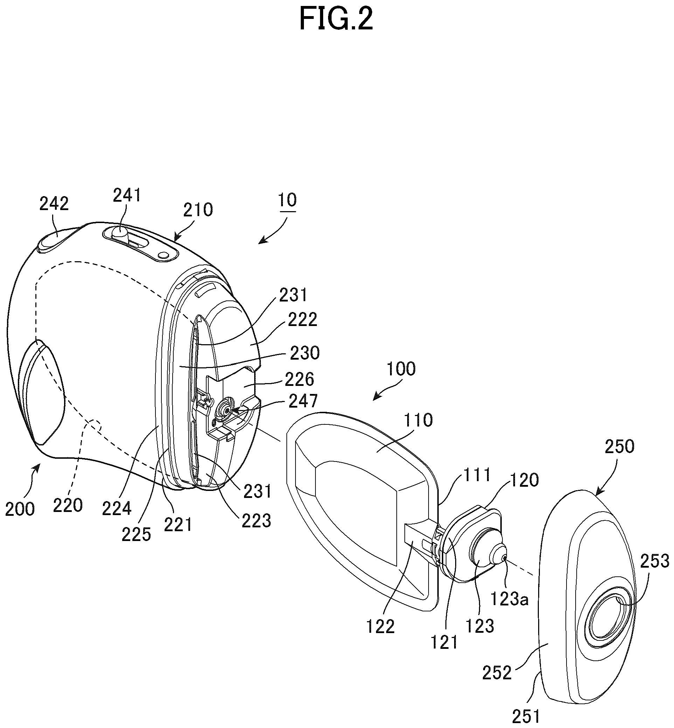

An electrostatic spray device 10 according to this embodiment, as illustrated in Fig. 1A, Fig. 1B, and Fig. 2, includes a cartridge 100 containing a liquid, and an electrostatic spray main body 200 for which the cartridge 100 can be inserted and removed. In addition, the electrostatic spray device 10 according to this embodiment further includes a cap 11 to be mounted on the electrostatic spray main body 200, in which the cap 11 is removed in use (refer to Fig. 1B), and the cap 11 is mounted after use (refer to Fig. 1A).

The electrostatic spray device 10 according to this embodiment is a hand-held type device which has a shape or a size that a user is capable of holding with a hand, and sprays a liquid composition (the liquid) toward a target by an electrostatic spray method. The electrostatic spray method is a method in which a high voltage (for example, several kV to several dozen kV) is applied to a liquid composition (for example, a solution in which a high-molecular compound is dissolved in a volatile solvent) such that the liquid composition is electrostatically charged, and the liquid composition is sprayed toward the target by an electrostatic force based on a potential difference between the electrically charged liquid composition and a target. The liquid composition sprayed by the electrostatic spray method is sent toward the target that is in the shape of an ultrafine yarn. In the sprayed liquid composition, the solvent that is a volatile substance is dried while the liquid composition is sprayed and then, is sent toward the target and after the liquid composition is attached to the target, and thus, a film can be formed on the surface of the target. Note that, the electrostatic spray device 10 according to this embodiment can also be used as an electrostatic spinning device spraying a solution containing a raw material for electrostatic spinning, that is, a spinning liquid toward the target.

For example, in a case where a solution containing a volatile substance, a water-insoluble polymer for forming a fiber, and water is adopted as the liquid composition, the user holds the electrostatic spray device 10 with the hand and sprays the liquid composition toward the skin of the user, and thus, a film can be formed on the surface of the skin of the user. The film is a deposited material containing a fiber.

As illustrated in Fig. 2, the electrostatic spray main body 200 includes a housing 210, and a cover 250 to be mounted on the housing 210. For the housing 210, the cartridge 100 and the cover 250 are attachable and detachable.

The housing 210 and the cover 250 are formed of an insulating material, that is, a material having properties of hardly conducting electricity. Note that, here, "insulating" or "hardly conducting electricity", for example, indicates having volume resistivity (ASTM D257, JIS K6911) of greater than 10¹² Ωm. For example, an insulating organic material such as a synthetic resin, an insulating inorganic material such as glass or ceramic, or the like is exemplified as the insulating material used in the housing 210 and the cover 250. For example, polypropylene (PP), polyacetal, polyether ether ketone (PEEK), polytetrafluoroethylene (PTFE), monomer cast nylon, and the like can be used as the insulating organic material. On the other hand, a conductive material indicates a material having properties of easily conducting electricity, that is, a material, for example, having volume resistivity of less than or equal to 10⁻² Ωm.

### [Configuration of Cover]

The cover 250 will be described with reference to Fig. 1B and Fig. 2. The cover 250 includes one opening 251 and the other opening 253, and is in a tubular shape in which an opening area is narrowed toward the other opening 253 from one opening 251. For this reason, one opening 251 is wider than the other opening 253. A portion along one opening 251 of the cover 250 is an abutting edge portion 252. Such an abutting edge portion 252 is fitted into a second engaging step 225 of the housing 210 described below, and thus, the cover 250 is mounted on the housing 210. In a case where the cover 250 is mounted on the housing 210 on which the cartridge 100 described below is mounted, a nozzle 123 of the cartridge 100 passes through the other opening 253 of the cover 250, and a spray hole 123a of the nozzle 123 is in a state of facing the outside of the cover 250. That is, the cover 250 is configured to allow the spray of the liquid composition from a spray unit 120 of the cartridge 100 even in the case of being mounted on the housing 210.

Even when the cover 250 is closely mounted on the housing 210, a minute gap may exist between the abutting edge portion 252 of the cover 250 and the second engaging step 225 formed on an attachable and detachable surface 222 of the housing 210 described below. That is, even when the cover 250 is closely mounted on the housing 210, a minute gap may exist in a boundary portion (a parting line portion) between the cover 250 and the housing 210.

### [Configuration of Cartridge]

The cartridge 100 is a disposable container to be exchangeably mounted on a device that is a supply target of the liquid, and a use application thereof is not particularly limited, but in this embodiment, the cartridge 100 is a cartridge for an electrostatic spinning device that is used in an electrostatic spinning device. Specifically, as illustrated in Fig. 2, the cartridge 100 includes a liquid containing pouch (a liquid containing portion) 110 that is capable of containing the liquid, and the spray unit 120 spraying the liquid in the liquid containing pouch 110.

The liquid containing pouch 110 is a flat pouch-shaped airtight container formed by stacking two sheet bodies having the same shape and the same size and by joining (in this embodiment, heat-sealing) the circumferential edge portions of both of the sheet bodies, and contains the liquid composition inside.

Each of the sheet bodies includes a laminated body including an inner layer that forms an inner surface at the time of being in the shape of a pouch, an outer layer that forms an outer surface, and a conductive layer that is disposed between the inner layer and the outer layer, the outer layer and the inner layer are formed to have a thickness that is thin enough to be conductive, and thus, the sheet bodies have conductivity. Note that, an arbitrary layer, for example, an adhesive layer such as a PET film, and the like may be further provided between the inner layer and the outer layer.

It is preferable that the inner layer is formed of a sealant layer that has resistance to the liquid composition to be contained and has a sealant effect (heat-sealing properties). Specifically, non-stretched polypropylene (CPP), low-density polyethylene (LDPE), linear low-density polyethylene (LLDPE), a blend of LDPE and LLDPE, medium-density polyethylene (MDPE), high-density polyethylene (HDPE), metallocene polyethylene, an ethylene vinyl acetate copolymerization film (EVA), and the like can be used. Among them, the low-density polyethylene (LDPE), the linear low-density polyethylene (LLDPE), and the blend of LDPE and LLDPE are preferable. The thickness of the inner layer is preferably greater than or equal to 30 µm, and is more preferably greater than or equal to 40 µm. In addition, the thickness of the inner layer is preferably less than or equal to 150 µm, and is more preferably less than or equal to 80 µm.

The outer layer is formed of a material having a mechanical strength that is more excellent than that of the inner layer. For example, polyethylene terephthalate (PET), stretched polypropylene (OPP), stretched nylon (ONy), and the like are exemplified as the material of the outer layer. Among them, the polyethylene terephthalate (PET) is preferable. The thickness of the outer layer is preferably greater than or equal to 10 µm, and is more preferably greater than or equal to 15 µm. In addition, the thickness of the outer layer is preferably less than or equal to 30 µm, and is more preferably less than or equal to 20 µm. The outer layer may include a printing layer on the interface with the conductive layer. In general, the printing layer is formed of an ink containing a coloring agent that is easily dissolved in a high-volatility solvent liquid composition as a main component.

The conductive layer is formed of a material having conductivity that is capable of applying a voltage applied from the outer layer side to the liquid composition through the inner layer. For example, an aluminum sheet can be used as the conductive layer. The thickness of the aluminum sheet is preferably greater than or equal to 5 µm, and is more preferably greater than or equal to 7 µm. The thickness of the aluminum sheet is preferably less than or equal to 15 µm, and is more preferably less than or equal to 9 µm.

In addition, a vapor-deposited film in which aluminum, aluminum oxide, conductive silica, indium tin oxide (ITO), zinc oxide (ZnO), tin dioxide (SnO₂), or the like is vapor-deposited on a resin film such as a PET film or a polypropylene (PP) film can also be used as the conductive layer. The thickness of the resin film is preferably greater than or equal to 10 µm, and is more preferably greater than or equal to 15 µm. The thickness of the resin film is preferably less than or equal to 30 µm, and is more preferably less than or equal to 20 µm.

The vapor-deposited film can be configured by suitably combining the resin film with the material of a vapor-deposited layer. In particular, it is preferable that the PET film is used as the resin film, and the aluminum is used as the vapor-deposited layer.

The conductive layer has volume resistivity that is capable of sufficiently applying a voltage to the liquid composition in the liquid containing pouch 110. Specifically, the conductive layer, for example, preferably has volume resistivity of less than or equal to 10⁻² Ωm, and more preferably has volume resistivity of less than or equal to 10⁻⁷ Ωm.

In addition, the conductive layer is provided over a range that is capable of sufficiently applying a voltage to the liquid composition in the liquid containing pouch 110. Specifically, the conductive layer preferably occupies a range of greater than or equal to 5%, more preferably occupies a range of greater than or equal to 10%, even more preferably occupies a range of greater than or equal to 30%, and is still even more preferably provided over the entire surface of the liquid containing pouch 110, with respect to the entire liquid containing pouch 110. The conductive layer is provided to occupy the range described above, and thus, even in a case where the residual amount of the liquid composition is reduced with the use, the liquid composition and the conductive layer can be prevented from being separated from each other, and therefore, a voltage can be stably applied to the liquid composition. In addition, in a case where the conductive layer is partially provided, it is preferable that a part (a conductive portion) thereof is provided at a containing location of the liquid composition, and it is preferable that the position thereof is closer to the spray unit 120 from the viewpoint of sufficiently applying a voltage to the liquid composition even in a case where the residual amount of the liquid composition is reduced with the use. Further, the conductive layer (the conductive portion) may be provided only on one surface of the liquid containing pouch 110, and it is preferable that the conductive layer is provided on both surfaces of the liquid containing pouch 110 from the viewpoint of reliably applying a voltage to the liquid composition even in a case where the residual amount of the liquid composition is reduced with the use.

The conductive layer may function as a barrier layer that prevents the liquid composition in the liquid containing pouch 110 from being infiltrated into the outer layer. As described above, the conductive layer functions as the barrier layer, and thus, the transpiration of the liquid composition (a volatile liquid solvent) can be suppressed, and therefore, the precipitation of a polymer or the like that is dissolved in the liquid composition can be suppressed, and the stability of the content can be effectively improved. In addition, in a case where the aluminum sheet or the vapor-deposited film is used in the conductive layer, the conductive layer itself has light blocking properties, and thus, the content can be effectively protected. Further, it is not necessary to impart the light blocking properties to the printing layer, and thus, the number of colors that can be used in printing can be increased. Further, the conductive layer has glossiness, and thus, design properties can be improved.

The liquid containing pouch 110 has flexibility such that a region inside the joined circumferential edge portion can be deformed in accordance with a volume change in the content (for example, a reduction in the amount of liquid composition that is contained). That is, the liquid containing pouch 110 includes the circumferential edge portion that is not deformed in accordance with the volume change in the content, and a flexible region that is deformable in accordance with the volume change in the content. When the liquid composition is sufficiently contained, the flexible region of the liquid containing pouch 110 is in a swollen shape that is swollen to the outside, but a swelling amount is gradually reduced as the liquid composition that is contained is reduced, and finally, the flexible region is in the shape of a flat surface. Note that, here, the "content" is a collective term of the liquid composition contained in the liquid containing pouch 110 and gas (the air) sealed inside the liquid containing pouch 110.

The liquid composition contained in the liquid containing pouch 110 is not particularly limited, and for example, a liquid composition for spinning that is used in the electrostatic spinning device is exemplified. In addition, for example, a solution in which a high-molecular compound that is capable of forming a fiber is dissolved in a solvent can be used as the liquid composition. Both of a water-soluble high-molecular compound and a water-insoluble high-molecular compound can be used as the high-molecular compound.

In the case of using the water-insoluble high-molecular compound, the liquid composition contains greater than or equal to 50 mass% of a volatile solution selected from alcohol and ketone. The volatile solution is a substance having volatility in a liquid state. A vapor pressure of the volatile solution at 20°C is preferably greater than or equal to 0.01 kPa and less than or equal to 106.66 kPa, is more preferably greater than or equal to 0.13 kPa and less than or equal to 66.66 kPa, is even more preferably greater than or equal to 0.67 kPa and less than or equal to 40.00 kPa, and is still even more preferably greater than or equal to 1.33 kPa and less than or equal to 40.00 kPa.

Among the volatile solutions, for example, monovalent chain aliphatic alcohol, monovalent cyclic aliphatic alcohol, and monovalent aromatic alcohol are preferably used as the alcohol. Examples of the monovalent chain aliphatic alcohol include C₁-C₆ alcohol, examples of the monovalent cyclic alcohol include C₄-C₆ cyclic alcohol, and examples of the monovalent aromatic alcohol include benzyl alcohol, and phenyl ethyl alcohol. Specific examples thereof include ethanol, isopropyl alcohol, butyl alcohol, phenyl ethyl alcohol, n-propanol, and n-pentanol. One type or two or more types selected from such alcohols can be used.

Among the volatile solutions, examples of the ketone include C₁-C₄ alkyl ketone such as acetone, methyl ethyl ketone, and methyl isobutyl ketone. Only one type of such ketones can be used or two or more types thereof can be used by being combined.

The volatile solution is more preferably one type or two or more types selected from ethanol, isopropyl alcohol, and butyl alcohol, is even more preferably one type or two or more types selected from ethanol and butyl alcohol, and is still even more preferably a volatile solution containing ethanol from the viewpoint of improving the touch of a fiber to be formed.

The content of the volatile solution in the liquid composition is preferably greater than or equal to 50 mass%, is more preferably greater than or equal to 55 mass%, and is even more preferably greater than or equal to 60 mass%. In addition, the content of the volatile solution in the liquid composition is preferably less than or equal to 95 mass%, is more preferably less than or equal to 94 mass%, and is even more preferably less than or equal to 93 mass%. The content of the volatile solution in the liquid composition is preferably greater than or equal to 50 mass% and less than or equal to 95 mass%, is more preferably greater than or equal to 55 mass% and less than or equal to 94 mass%, and is even more preferably greater than or equal to 60 mass% and less than or equal to 93 mass%. By containing the volatile solution in the liquid composition at such a ratio, the liquid composition can be sufficiently volatilized at the time of performing the electrostatic spray method, and a film containing a fiber on the surface of the skin or the nail can be formed.

In addition, the ethanol is preferably greater than or equal to 50 mass%, is more preferably greater than or equal to 65 mass%, and is even more preferably greater than or equal to 80 mass%, with respect to the total amount of the volatile solution, from the viewpoint of high volatility and the touch of the fiber to be formed. In addition, the ethanol is preferably less than or equal to 100 mass%. The ethanol is preferably greater than or equal to 50 mass% and less than or equal to 100 mass%, is more preferably greater than or equal to 65 mass% and less than or equal to 100 mass%, and is even more preferably greater than or equal to 80 mass% and less than or equal to 100 mass%, with respect to the total amount of the volatile solution.

In addition, it is preferable that the liquid composition contains the water-insoluble polymer for forming a fiber. The water-insoluble polymer for forming a fiber is a substance that can be dissolved in the volatile solution. Here, being dissolved indicate that the water-insoluble polymer for forming a fiber is in a dispersed state at 20°C and the dispersed state is a visually homogeneous state, preferably, a visually transparent or semi-transparent state.

The water-insoluble polymer for forming a fiber is a polymer that is soluble in the volatile substance and is insoluble in water. Herein, a "water-soluble polymer" indicates a water-soluble polymer having properties in which 1 g of the polymer is weighed, and then, is dipped in 10 g of ion exchange water, and greater than or equal to 0.5 g of the dipped polymer is dissolved in water after 24 hours elapse, in an environment of one atmosphere and 23°C. On the other hand, herein, the "water-insoluble polymer" indicates a water-insoluble polymer having properties in which 1 g of the polymer is weighed, and then, is dipped in 10 g of ion exchange water, and greater than or equal to 0.5 g of the dipped polymer is not dissolved after 24 hours elapse, in other words, a dissolution amount is less than 0.5 g, in an environment of one atmosphere and 23°C.

Examples of the polymer that is insoluble in water and has fiber forming ability include completely saponified polyvinyl alcohol that can be subjected to an insoluble treatment after forming a film, partially saponified polyvinyl alcohol that can be subjected to a cross-linking treatment after forming a film by being used together with a cross-linking agent, oxazoline modified silicone such as a poly(N-propanoyl ethylene imine) graft-dimethyl siloxane/γ-aminopropyl methyl siloxane copolymer, polyvinyl acetal diethyl aminoacetate, zein (a main component of corn protein), polyester, a polylactic acid (PLA), an acrylic resin such as a polyacrylonitrile resin and a polymethacrylate resin, a polystyrene resin, a polyvinyl butyral resin, a polyethylene terephthalate resin, a polybutylene terephthalate resin, a polyurethane resin, a polyamide resin, a polyimide resin, and a polyamide imide resin. One type or two or more types selected from such water-insoluble polymers can be used by being combined. Among such water-insoluble polymers, it is preferable to use one type or two or more types selected from the completely saponified polyvinyl alcohol that can be subjected to the insoluble treatment after forming the film, the partially saponified polyvinyl alcohol that can be subjected to the cross-linking treatment after forming the film by being used together with the cross-linking agent, the polyvinyl butyral resin, the polyurethane resin, the acrylic resin such as the polymethacrylate resin, the polyvinyl acetal diethyl aminoacetate, the oxazoline modified silicone such as the poly(N-propanoyl ethylene imine) graft-dimethyl siloxane/γ-aminopropyl methyl siloxane copolymer, the polylactic acid (PLA), and zein. Among such water-insoluble polymers, the partially saponified polyvinyl alcohol, the completely saponified polyvinyl alcohol, the polyvinyl butyral resin, the polymethacrylate resin, and the polyurethane resin are more preferable, and the partially saponified polyvinyl alcohol, the completely saponified polyvinyl alcohol, and the polyvinyl butyral resin are even more preferable, from the viewpoint of dispersibility with respect to an alcohol solvent, the touch of the fiber, and the like, and the polyvinyl butyral resin is especially preferable from the viewpoint of being capable of stably and efficiently forming the film containing the fiber on the surface of the skin or the nail and from the viewpoint of the durability of the film, film forming properties, and compatibility between following properties with respect to the skin and the durability.

The content of the water-insoluble polymer for forming a fiber in the liquid composition is preferably greater than or equal to 1 mass%, is more preferably greater than or equal to 3 mass%, and is even more preferably greater than or equal to 5 mass%. In addition, content of the water-insoluble polymer for forming a fiber in the liquid composition is preferably less than or equal to 30 mass%, is more preferably less than or equal to 25 mass%, and is even more preferably less than or equal to 20 mass%. The content of the water-insoluble polymer for forming a fiber in the liquid composition is preferably greater than or equal to 1 mass% and less than or equal to 30 mass%, is more preferably greater than or equal to 3 mass% and less than or equal to 25 mass%, and is even more preferably greater than or equal to 5 mass% and less than or equal to 20 mass%. By containing the water-insoluble polymer for forming a fiber in the liquid composition at such a ratio, a fibrous film can be stably and efficiently formed.

In addition, it is preferable that the liquid composition contains water. The water is ionized and charged compared to a solvent such as ethanol that is not ionized, and thus, is capable of imparting conductivity to the liquid composition. For this reason, a fibrous film is stably formed on the surface of the skin or the nail by electrostatic spray. In addition, the water contributes to the improvement of the adhesiveness of a film to be formed by the electrostatic spray with respect to the skin or the nail, the improvement of durability, and the appearance. The content of the water in the liquid composition is preferably greater than or equal to 0.2 mass% and less than or equal to 25 mass%, and is more preferably greater than or equal to 0.3 mass% and less than or equal to 20 mass%, from the viewpoint of obtaining such function effects, and is even more preferably greater than or equal to 0.4 mass% and less than or equal to 10 mass%, from the viewpoint of the forming properties of the fibrous film even in a humid environment.

The liquid composition may further contain other components. Examples of the other components include polyol, liquid oil, a plasticizer of the water-insoluble polymer for forming a fiber, an electroconductivity control agent of the liquid composition, a water-soluble polymer, a powder such as a coloring pigment and an extender pigment, a colorant, a perfume, a repellent, an oxidant inhibitor, a stabilizer, an antiseptic agent, and various vitamins. In a case where the other components are contained in the liquid composition, a content ratio of the other components is preferably greater than or equal to 0.1 mass% and is less than or equal to 30 mass%, and is more preferably greater than or equal to 0.5 mass% and less than or equal to 20 mass%.

Further, glycol can be contained in the liquid composition. Examples of the glycol include ethylene glycol, propylene glycol, butylene glycol, diethylene glycol, dipropylene glycol, and polypropylene glycol. From the viewpoint of sufficiently volatilizing the volatile solution at the time of performing the electrostatic spray method, from the viewpoint of fiber forming properties, and from the viewpoint of the touch of the fiber to be formed, the content of the glycol in the liquid composition is preferably less than or equal to 10 mass%, is more preferably less than or equal to 3 mass%, and is even more preferably less than or equal to 1 mass%, and it is preferable that the glycol is not substantially contained.

The viscosity of the liquid composition at 25°C is preferably greater than or equal to 2 mPa·s and less than or equal to 3000 mPa·s, is more preferably greater than or equal to 10 mPa·s and less than or equal to 1500 mPa·s, is even more preferably greater than or equal to 15 mPa·s and less than or equal to 1000 mPa·s, and is still even more preferably greater than or equal to 15 mPa·s and less than or equal to 800 mPa·s, from the viewpoint of stably forming the fibrous film, from the viewpoint of spinning properties at the time of performing the electrostatic spray, from the viewpoint of improving the durability of the film, and from the viewpoint of improving the touch of the film. The viscosity of the liquid composition is measured at 25°C by using an E-type viscosimeter. For example, an E-type viscosimeter (VISCONICEMD) manufactured by TOKYO KEIKI INC. can be used as the E-type viscosimeter. As a measurement condition of such a case, 25°C is set, a cone-plate rotor No. 43 is used, a suitable number of rotations according to the viscosity is selected as the number of rotations, and the number of rotations is 5 rpm in a case where the viscosity is greater than or equal to 500 mPa·S, is 10 rpm in a case where the viscosity is greater than or equal to 150 mPa·S and less than 500 mPa·S, and is 20 rpm in a case where the viscosity is less than 150 mPa·S.

The spray unit 120 includes a mounting body 121, a connection body 122, and the nozzle 123, and in this embodiment, the spray unit 120 is formed of a resin.

The mounting body 121 includes a flow path and a pump (for example, a gear pump) inside. The flow path is a passage that circulates the liquid composition. The pump is disposed in the middle of the flow path, and is driven to suck the liquid composition to be circulated in the flow path. The details of the flow path or the pump will be described below.

The connection body 122 is mechanically connected to the liquid containing pouch 110 and is communicated with the inside of the liquid containing pouch 110, and guides the liquid composition in the liquid containing pouch 110 to the flow path of the mounting body 121. The nozzle 123 is integrally formed with the mounting body 121, and has a spray hole 123a on the tip and has a nozzle flow path that connects the spray hole 123a and the flow path of the mounting body 121. The details will be described below, but the electrically charged liquid composition passes through the nozzle flow path formed in the nozzle 123, and then, is sprayed from the spray hole 123a.

### [Internal Configuration of Mounting Body of Spray Unit]

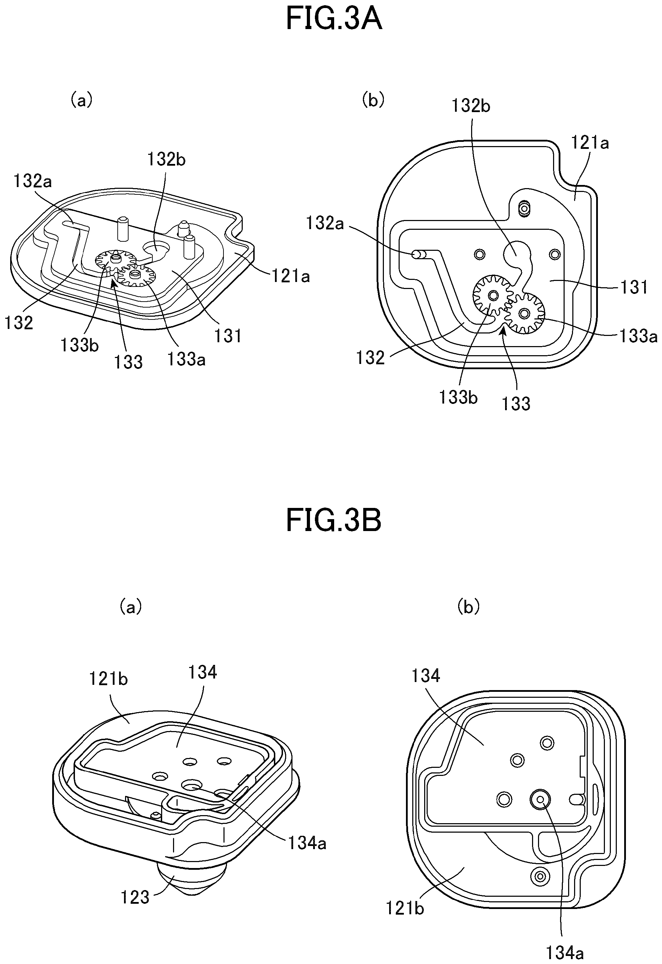

The internal configuration of the mounting body 121 of the spray unit 120 will be described with reference to Figs. 3A(a) and 3A(b) and Figs. 3B(a) and 3B(b). Figs. 3A(a) and 3A(b) illustrate the inner surface of one member 121a when the mounting body 121 is exploded, and the connection body 122 is disposed on the outer surface side of the member 121a (the surface on the lower side of Fig. 3A(a)). Figs. 3B(a) and 3B(b) illustrate the inner surface of the other member 121b when the mounting body 121 is exploded, and the nozzle 123 is disposed on the outer surface side of the member 121b (the surface on the lower side of Fig. 3B(a)).

The member 121a and the member 121b are formed of an insulating resin. Then, the inner surface side of the member 121a and the inner surface side of the member 121b are closely joined, and thus, an outer shell housing of the mounting body 121 is configured.

As illustrated in Figs. 3A(a) and 3A(b), a flow path forming plate (a flow path forming member) 131 is provided on the inner surface of one member 121a. A flow path groove 132 is formed in the flow path forming plate 131. An inflow end 132a of the flow path groove 132 is connected to the liquid containing pouch 110 through the connection body 122. An ejection end 132b of the flow path groove 132 is a portion that is connected to a hole 134a of the other member 121b (refer to Figs. 3B(a) and 3B(b)). The gear pump 133 including gears 133a and 133b is disposed in the middle of the flow path groove 132.

As illustrated in Figs. 3B(a) and 3B(b), a sealing plate (a flow path forming member) 134 is provided on the inner surface of the other member 121b. In the sealing plate 134, a hole 134a that leads to the nozzle 123 is formed in a position corresponding to the ejection end 132b of the flow path forming plate 131. In a case where the inner surface side of the member 121a and the inner surface side of the member 121b are closely joined, the sealing plate 134 is closely attached to the flow path forming plate 131 and seals the flow path groove 132. Accordingly, a flow path along the flow path groove 132 is formed. For this reason, the liquid composition flowing from the inflow end 132a is capable of reaching the ejection end 132b by being circulated in the flow path (the flow path groove 132), and is capable of flowing in the nozzle 123 through the hole 134a.

The flow path forming plate 131, the gear pump 133, and the sealing plate 134 contain a material having resistance to the liquid composition contained in the liquid containing pouch 110. On the other hand, in the electrostatic spray device 10 according to this embodiment, as described below, the liquid composition is electrostatically charged by applying a voltage to the liquid containing pouch 110 without applying a voltage to the spray unit 120 of the cartridge 100. For this reason, it is not necessary to add a functional agent that imparts conductivity, such as carbon, to a resin material configuring the spray unit 120 (in particular, a resin material configuring the flow path forming plate 131, the gear pump 133, and the sealing plate 134). That is, the spray unit 120 can be formed of an insulating resin (a so-called carbonless resin). Note that, the "insulating resin" indicates a general resin that does not contain a conductive material such as a metal or carbon, has high electric resistance, and hardly allows electricity to flow, and for example, indicates a resin having volume resistivity of greater than or equal to 10¹² Ωm.

In this embodiment, the flow path forming plate 131, the gear pump 133, and the sealing plate 134 are formed of a single insulating resin having solvent resistance with respect to a solvent such as ethanol, such as a polypropylene (PP) resin, polyethylene terephthalate (PET), a polyethylene (PE) resin, and a polyacetal (POM) resin.

The flow path forming plate 131, the gear pump 133, and the sealing plate 134 are formed of such a single insulating resin having solvent resistance, and thus, the flow path forming plate 131, the gear pump 133, and the sealing plate 134 are rarely swollen or deformed even in contact with the solvent such as ethanol. For this reason, in the case of using a liquid composition in which ethanol is adopted as the solvent, for example, a liquid composition in which a polyvinyl butyral (PVB) resin is dissolved in ethanol, excellent operability of the device including the gear pump 133 can be ensured. In addition, the manufacturing cost can be decreased, compared to the case of using a conductive resin (for example, a resin containing carbon).

Note that, in this embodiment, the insulating resin (a so-called carbonless resin) is used in the flow path forming plate 131, but the present invention is not limited thereto, and a conductive resin (for example, a resin containing carbon) can also be adopted. Note that, the "conductive resin" indicates a resin that contains a conductive material such as a metal or carbon, has low electric resistance, and easily allows electricity to flow, and for example, indicates a resin having volume resistivity of less than or equal to 10⁻² Ωm. In such a case, for example, a technology can be adopted in which the liquid composition flowing in the flow path (the flow path groove 132) is electrically charged in the mounting body 121. In addition, in this case, a voltage is applied to the liquid containing pouch 110 and the flow path forming plate 131 to have the same potential, and thus, the potential is homogenized, and a voltage can be more sufficiently and stably applied to the liquid composition. Note that, as the configuration in which the liquid containing pouch 110 and the flow path forming plate 131 have the same potential, for example, a configuration is exemplified in which the liquid containing pouch 110 and the flow path forming plate 131 are electrically connected, and a high voltage is applied to the flow path forming plate 131 through the liquid containing pouch 110, but the present invention is not limited thereto.

### [External Configuration of Housing]

The housing 210 illustrated in Fig. 1A, Fig. 1B, and Fig. 2 has a shape and a size that the user is capable of holding with a hand. Specifically, the housing 210 has a flat shape, that is, a shape of which the cross-section orthogonal to a nozzle axis (an axis along a direction in which the liquid composition is sprayed from the nozzle 123) has a long axis and a short axis. In addition, the length of the housing 210 in a direction along the nozzle axis, for example, is greater than or equal to 3 cm and less than or equal to 11 cm.

In the housing 210, a containing space 220 that is capable of containing the liquid containing pouch 110 of the cartridge 100 (refer to Fig. 2) is formed, and an electric-charging structure for electrostatically charging the liquid composition, a driving unit for driving the pump provided in the mounting body 121, or the like is disposed. The details of the electric-charging structure, the driving unit, or the like will be described below.

Note that, herein, in the surfaces of the housing 210, the surface on which the cover 250 is mounted will be referred to as the "attachable and detachable surface 222". In addition, in the attachable and detachable surface 222, an outer circumferential edge portion that is in contact with the abutting edge portion 252 of the cover 250 will be referred to as a "circumferential edge 221". Hereinafter, the structure of the attachable and detachable surface 222 will be described with reference to Fig. 2.

As illustrated in Fig. 2, the attachable and detachable surface 222 is approximately an elliptical surface that is surrounded by the circumferential edge 221 in an elliptical shape. An insertion hole 223 is formed in the attachable and detachable surface 222 in a state of being approximately parallel to a long axis of the ellipse. The insertion hole 223 is communicated with the containing space 220 in the housing 210, and has a shape and a size in which the liquid containing pouch 110 of the cartridge 100 can be inserted and removed in the containing space 220 through the insertion hole 223. In this embodiment, the insertion hole 223 and the containing space 220 are formed in a position closer to one portion of the circumferential edge 221 but not in the central position of the ellipse, and thus, a large space for containing the electric-charging structure, the driving unit, or the like can be ensured on the other portion side of the circumferential edge 221.

In the attachable and detachable surface 222, a first engaging step 224 along the circumferential edge 221 is formed, and a second engaging step 225 is formed on the inner circumference side of the first engaging step 224. An opening edge portion 11a (refer to Fig. 1B) is fitted into the first engaging step 224, and thus, the cap 11 is mounted to be attachable and detachable with respect to the housing 210. The abutting edge portion 252 (refer to Fig. 2) that is a portion along one opening 251 is fitted into the second engaging step 225, and thus, the cover 250 is mounted to be attachable and detachable with respect to a portion along the circumferential edge 221 of the housing 210.

A mounting recess 226 is formed on the attachable and detachable surface 222. The mounting recess 226 is in a shape in which the mounting body 121 is retained in a positioned state when the mounting body 121 of the cartridge 100 is fitted into the mounting recess 226. For this reason, in a case where the liquid containing pouch 110 of the cartridge 100 is inserted to the containing space 220 through the insertion hole 223, and the mounting body 121 is closely fitted into the mounting recess 226 such that the position is retained, the cartridge 100 is mounted accurately in the designated position of the housing 210.

An antistatic wall 230 is erected on the attachable and detachable surface 222. In this embodiment, the antistatic wall 230 is formed of a resin that is an insulating material integrally with the housing 210. Note that, the details of a disposition position, the structure, or the like of the antistatic wall 230 will be described below.

### [Internal Configuration and Operation of Housing]

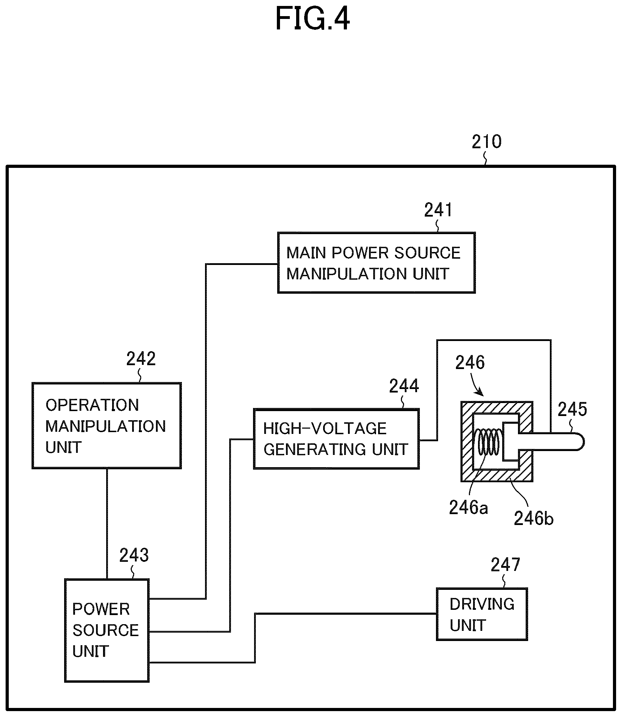

The electric-charging structure, the driving unit, or the like that is provided in the housing 210 will be described with reference to Fig. 4. A main power source manipulation unit 241 and an operation manipulation unit 242 are attached to the housing 210 in a state where the main power source manipulation unit 241 and the operation manipulation unit 242 can be manipulated from the outside of the housing 210. A power source unit 243, a high-voltage generating unit 244, a contact portion 245, a biasing portion 246, and a driving unit 247 are provided inside the housing 210. Note that, the field electrode covering the entire liquid containing pouch (liquid containing portion) in which the liquid composition is contained is not disposed.

The contact portion 245 has conductivity that is capable of conveying a high voltage from the high-voltage generating unit 244 to a contact target, and when the liquid containing pouch 110 of the cartridge 100 is contained in the designated position of the containing space 220 of the housing 210, the tip surface of the contact portion 245 is disposed in a position in contact with the liquid containing pouch 110. That is, the contact portion 245 is configured such that the contact portion 245 is in contact with a portion having conductivity of the liquid containing pouch 110 contained in the electrostatic spray main body 200, and thus, the high voltage from the high-voltage generating unit 244 can be applied to the liquid in the liquid containing pouch 110 through the portion having conductivity. Note that, even in a case where the outer layer and the inner layer of the liquid containing pouch 110 are formed of a non-conductive material, insofar as the thickness of the outer layer and the inner layer is thin, and thus, it is possible to conduct electricity to the conductive layer that is an intermediate layer through the outer layer and to the liquid composition through the inner layer, the outer layer, the conductive layer, and the inner layer can be collectively evaluated as the "portion having conductivity of the liquid containing pouch 110".

It is preferable that the contact portion 245 is disposed in a position in contact with the circumferential edge portion but not the flexible region of the liquid containing pouch 110 (the central wide surface portion), from the viewpoint of suppressing liquid leakage due to a pressure on the flexible region (central wide surface portion). In addition, the present invention is not limited thereto, and when the cartridge 100 is mounted on the housing 210, the contact portion 245 may be disposed in a position in contact with the flexible region (the central wide surface portion), from the viewpoint of preventing a contact failure due to the deformation of the circumferential edge portion of the liquid containing pouch 110. In the contact portion 245, an arbitrary shape can be adopted, and it is preferable that the contact portion 245 is formed of a columnar conductive material having a tapered surface that is inclined along an insertion direction of the liquid containing pouch 110, for example, having a rounded tip surface.

It is preferable that a contact area of the contact portion 245 with respect to the liquid containing pouch 110 is greater than or equal to 2 mm², from the viewpoint of preventing the break or the like of the liquid containing pouch 110 by reliable contact.

The biasing portion 246 includes a biasing member 246a biasing the contact portion 245 against the containing space 220 side of the housing 210 and includes a guide structure 246b allowing and guiding a back-and-forth movement operation of the contact portion 245. The biasing portion 246, for example, is formed of an elastic member such as a coil spring.

The contact portion 245 is biased to the containing space 220 side, that is, in a direction of being pressed against the liquid containing pouch 110 contained in the containing space 220 by a small force at which the liquid composition in the liquid containing pouch 110 is not leaked through the nozzle 123, by the biasing portion 246. For this reason, the contact portion 245 performs the following movement operation, in accordance with the state of the liquid containing pouch 110.
(1) When the liquid containing pouch 110 of the cartridge 100 is inserted to the containing space 220 through the insertion hole 223 of the housing 210. At this time, the contact portion 245 is pressed in a direction opposite to a biasing direction by the liquid containing pouch 110 that is inserted, and thus, performs the back movement while retaining the contact with respect to the liquid containing pouch 110.
(2) When the liquid containing pouch 110 of the cartridge 100 is contained in the designated position of the containing space 220 of the housing 210. At this time, the contact portion 245 is in contact with the circumferential edge portion or the flexible region (the central wide surface portion) of the liquid containing pouch 110.
(3) When the swelling amount is gradually reduced from a state of being swollen to the outside as the liquid composition is used as the liquid composition is used, and thus, the liquid containing pouch 110 of the cartridge 100 is in the shape of a flat surface in a case where the contact portion 245 is in contact with the flexible region (the central wide surface portion) of the liquid containing pouch 110. At this time, the contact portion 245 performs the forth movement in the biasing direction, in accordance with the deformation of the liquid containing pouch 110, and retains the contact with respect to the liquid containing pouch 110.

An operation that is performed by manipulating the main power source manipulation unit 241 and the operation manipulation unit 242 will be described. As the premise of the operation, the following states of (a), (b), and (c) are assumed. Note that, in general use, the state of (c) is set, but even in a case where the state of (c) is not set, the operation based on the manipulation of the main power source manipulation unit 241 and the operation manipulation unit 242 can be performed.
(a) The liquid containing pouch 110 of the cartridge 100 is contained in the containing space 220 through the insertion hole 223, and the cartridge 100 is mounted accurately in the designated position of the housing 210.
(b) According to (a) described above, the contact portion 245 is in contact with the conductive liquid containing pouch 110.
(c) The cover 250 is mounted on the housing 210 (refer to Fig. 1B).

In a case where the state of (a) described above is set, the driving unit 247 is mechanically connected to the gear 133a of the gear pump 133 provided in the mounting body 121 of the cartridge 100. In addition, in a case where the state of (b) described above is set, the high-voltage generating unit 244 and the liquid containing pouch 110 can be electrically connected through the contact portion 245.

In a case where the main power source manipulation unit 241 is turned off (OFF), the power is not supplied to the high-voltage generating unit 244 or the driving unit 247 from the power source unit 243. For this reason, a high voltage is not generated from the high-voltage generating unit 244, and the driving unit 247 is not driven. Therefore, in a case where the main power source manipulation unit 241 is turned OFF, the liquid composition contained in the liquid containing pouch 110 is not electrostatically charged and the liquid composition is not sprayed even when the user mistakenly manipulates the operation manipulation unit 242.

The operation manipulation unit 242, for example, includes a switch that is capable of switching an on (ON) state and an off (OFF) state.

In a case where the operation manipulation unit 242 is turned ON when the main power source manipulation unit 241 is in the ON state, the power is supplied to the high-voltage generating unit 244 and the driving unit 247 from the power source unit 243. Then, the driving unit 247 is driven, a rotative force is generated, the rotative force is transferred to the gear 133a of the gear pump 133 provided in the mounting body 121 of the cartridge 100, and the gear pump 133 is driven. The liquid composition in the liquid containing pouch 110 is sucked to the spray unit 120 side by the driving of the gear pump 133. In addition, the high-voltage generating unit 244 generates a positive high voltage (for example, several kV to several dozen kV), and sends the generated high voltage to the contact portion 245. The high voltage is applied to the conductive liquid containing pouch 110 that is in contact with the contact portion 245 through the contact portion 245, and thus, the liquid composition contained in the liquid containing pouch 110 is electrostatically charged.

That is, the conductive liquid containing pouch 110 has not only a function of containing the liquid composition but also a function as an electrode for electrostatically charging the liquid composition. However, the liquid containing pouch 110 is large enough to contain the liquid composition and is directly in contact with the liquid composition, and thus, is capable of effectively and electrostatically charging the liquid composition. As described above, the liquid composition is electrostatically charged by using the conductive liquid containing pouch 110, and thus, a large electrode is not necessary, the housing 210 can be downsized, and the electrostatic spray device 10 can be downsized.

In a case where the liquid composition that is electrostatically charged as described above flows in the spray unit 120 from the liquid containing pouch 110 and reaches the spray hole 123a of the nozzle 123, the liquid composition is sprayed toward the target by the electrostatic force based on the potential difference between the electrically charged liquid composition and the target. After that, in a case where the operation manipulation unit 242 is turned OFF, the spray of the liquid composition is stopped.

In the housing 210, a selector switch (not illustrated) that is capable of adjusting a spray amount of the liquid composition in a multistage (for example, three stages of large/medium/small) may be provided, in addition to the main power source manipulation unit 241 and the operation manipulation unit 242.

In the example described above, the operation manipulation unit 242 is a switch for selecting only two states of ON/OFF, but is not limited thereto, and for example, may be a switch that is turned on (ON) by being pressed, is returned to off (OFF) by stopping the pressing, and is capable of changing a pressing amount in the ON state. In a case where the operation manipulation unit 242 is the switch that is capable of changing the pressing amount in the ON state, the spray amount of the liquid composition to be sprayed from the nozzle 123 can be adjusted in accordance with a change in the pressing amount. That is, in a case where the pressing amount of the operation manipulation unit 242 is increased, the power to be supplied to the driving unit 247 from the power source unit 243 increases, and the number of rotations of the driving unit 247 increases. Then, the number of rotations of the gear pump 133 provided in the mounting body 121 increases, the amount of liquid composition that is sucked to (flows in) the spray unit 120 side from the liquid containing pouch 110 increases, and the spray amount of the liquid composition to be sprayed also increases. On the other hand, in a case where the pressing amount of the operation manipulation unit 242 decreases, the voltage to be supplied from the power source unit 243 to the driving unit 247 decreases, and the number of rotations of the driving unit 247 decreases. Then, the number of rotations of the gear pump 133 provided in the mounting body 121 decreases, the amount of liquid composition that is sucked to (flows in) the spray unit 120 side from the liquid containing pouch 110 decreases, and the spray amount of the liquid composition to be sprayed also decreases.

In the embodiment described above, the contact portion 245 formed of the columnar conductive material having the rounded tip surface is adopted, but a plate spring-shaped contact portion can also be adopted. In this case, the plate spring-shaped contact portion may be disposed such that the plate spring-shaped contact portion is in contact with the circumferential edge portion of the liquid containing pouch 110 (a portion in which two thin-layer aluminum films are joined), that is, a portion of the liquid containing pouch 110 that is rarely deformed regardless of a containing amount of the liquid composition. The plate spring-shaped contact portion is biased to the liquid containing pouch 110 side by the own spring force, and thus, an excellent contact state can be retained, and a separate member for biasing is not necessary, and thus, a device configuration can be simplified.

In addition, in the embodiment described above, the liquid containing pouch 110 has conductivity over the entire surface, but a liquid containing pouch can also be adopted in which at least a portion in contact with the contact portion 245 has conductivity. For example, a configuration may be set in which only one surface of the liquid containing pouch 110 may have conductivity, in this case, a configuration may be set in which the entire other surface or a part thereof may have transparency such that the inside is visible. According to such a configuration in which the inside is visible, the residual amount of the liquid composition in the liquid containing pouch 110 can be visually checked.

Further, in the embodiment described above, the liquid containing pouch 110 is formed of the thin-layer aluminum film and has conductivity, but even in a case where the liquid containing pouch 110 is formed of the other conductive material, the present invention can be applied. In addition, a hard case container using a hard and conductive resin may be adopted instead of the liquid containing pouch 110. Further, a cylindrical liquid containing portion having conductivity can also be adopted instead of the liquid containing pouch 110. In the cylindrical liquid containing portion, the liquid composition is extruded from the liquid containing portion, and thus, the gear pump 133 may not be used.

### [Configuration and Function of Antistatic Wall]

The antistatic wall 230 will be described with reference to Fig. 2. The antistatic wall 230 is formed of an insulating resin integrally with the housing 210, and is erected on the attachable and detachable surface 222 of the housing 210. The antistatic wall 230 is disposed in a position between the insertion hole 223 and the circumferential edge 221 on the attachable and detachable surface 222, and extends along the circumferential edge 221. More specifically, the antistatic wall 230 is disposed in a position between the insertion hole 223 and a portion of the circumferential edge 221 close to the insertion hole 223. In this embodiment, the antistatic wall 230 is disposed exactly in a position in which the insertion hole 223 and the circumferential edge 221 are separated from each other.

Here, the height of the antistatic wall 230 will be described. In this description, in the liquid containing pouch 110 of the cartridge 100, a portion close to the spray unit 120 will be referred to as a shoulder portion 111 (refer to Fig. 2). In addition, the "height" indicates a dimension in a direction toward the cover 250 side from the housing 210 side (the attachable and detachable surface 222 side), in a state where the cover 250 is mounted on the housing 210.

In a state where the liquid containing pouch 110 of the cartridge 100 is contained in the containing space 220, and the cartridge 100 is mounted accurately in the designated position of the housing 210, the height of the antistatic wall 230 is higher than that of the shoulder portion 111 of the liquid containing pouch 110. The limit of the height of the antistatic wall 230 is a height at which the antistatic wall 230 is not in contact with the cover 250, in a state where the cover 250 is mounted on the housing 210.

A groove 231 extending along the circumferential edge 221 is formed in the tip surface of the antistatic wall 230. In this embodiment, two grooves 231 are linearly disposed in line.

As described above, the antistatic wall 230 is provided in the position between the insertion hole 223 and the circumferential edge 221, and thus, an insulating distance (a creeping distance) between the insertion hole 223 and the circumferential edge 221 (in particular, the portion of the circumferential edge 221 close to the insertion hole 223) increases by the existence of the antistatic wall 230. That is, the insulating distance between the insertion hole 223 and the circumferential edge 221 increases by a rising distance and a falling distance of the antistatic wall 230 (a distance twice the height of the antistatic wall 230), a distance for the thickness of the antistatic wall 230, and the insulating distance approximately corresponding to the falling distance and the rising distance of the groove 231 (a distance twice the depth of the groove 231).

When the electrostatic spray device 10 is used by mounting the cartridge 100 on the housing 210 and by mounting the cover 250 on the housing 210, the liquid containing pouch 110 having a high potential due to the high voltage that is applied from the contact portion 245 or the electrically charged liquid composition exists in the vicinity of the insertion hole 223. At this time, even in a case where a minute gap exists between the abutting edge portion 252 of the cover 250 and the second engaging step 225 of the housing 210, that is, in the boundary portion (the parting line portion) between the cover 250 and the housing 210, the insulating distance between the insertion hole 223 and the circumferential edge 221 is long, and thus, the electricity of the high voltage can be prevented from being leaked to the outside through the gap.

Further, the height of the antistatic wall 230 is higher than that of the shoulder portion 111 of the liquid containing pouch 110, and the electricity of the high voltage can be reliably prevented from being leaked to the outside through the gap. That is, a phenomenon in which the electricity of the high voltage is leaked to the outside from the liquid containing pouch 110 having a high potential or the electrically charged liquid composition through the gap, that is, a discharge phenomenon includes creeping discharge in which discharge proceeds along the surface of an insulator and space discharge in which discharge proceeds over the space. At this time, the height of the antistatic wall 230 is higher than that of the shoulder portion 111 of the liquid containing pouch 110, and thus, the space discharge can be reliably prevented. It is obvious that the creeping discharge can also be prevented by an increase in the height of the antistatic wall 230.

Eventually, the antistatic wall 230 is erected in the position between the insertion hole 223 and the circumferential edge 221, and thus, excellent discharge prevention performance can be ensured.

As described above, the electricity of the high voltage is not leaked to the outside from the liquid containing pouch 110 having a high potential due to the applied high voltage, or the like, and thus, the user is capable of comfortably using the electrostatic spray device 10 without feeling electric stimulation.

Further, a case in which the entire electrostatic spray device 10 is in a flat shape, as with this embodiment, from the viewpoint of improving the design will be described. In this case, the attachable and detachable surface 222 is an elliptical surface of which the flatness is emphasized, and as a result thereof, a configuration is set in which the insertion hole 223 and the circumferential edge 221 are close to each other (refer to Fig. 2). Even at this time, the antistatic wall 230 is disposed in the position between the insertion hole 223 and the circumferential edge 221, and thus, the electricity of the high voltage can be prevented from being leaked to the outside. In other words, the antistatic wall 230 is provided, and thus, the entire electrostatic spray device 10 can be in the flat shape, and the design properties can be improved.

Note that, in this embodiment, the groove 231 is formed in the tip surface of the antistatic wall 230, but a groove extending along the circumferential edge 221 may be formed in a rising surface of the antistatic wall 230 (a surface on the insertion hole 223 side) or a falling surface (a surface on the circumferential edge 221 side). Further, a convex portion extending along the circumferential edge 221 is formed in the antistatic wall 230, instead of the groove 231 or together with the groove 231, and thus, the insulating distance between the insertion hole 223 and the circumferential edge 221 can be increased.

The groove 231 or the convex portion is provided, and the insulating distance between the insertion hole 223 and the circumferential edge 221 is increased, and thus, the discharge prevention performance can be further improved, and a compact configuration can be obtained by decreasing the height of the antistatic wall 230 while ensuring the discharge prevention performance. Note that, a configuration can be set in which the groove 231 or the convex portion is not provided in the antistatic wall 230.

As described above, the preferred embodiment of the present invention has been described as an example, but the technical scope of the present invention is not limited to the scope described in the above embodiment. Various changes or improvements can be made in the embodiment described above.

Regarding the above-described embodiment, the present invention also includes the following electrostatic spinning device.
<1> An electrostatic spray device, comprising: an electrostatic spray main body capable of containing a liquid containing portion of a cartridge, the cartridge including the liquid containing portion which contains a liquid and a spray unit which sprays the liquid, the electrostatic spray device spraying the liquid by the electrostatic spray main body applying a voltage to the liquid, the cartridge having conductivity in at least one portion of the liquid containing portion, and the electrostatic spray main body including a high-voltage generating unit which generates a high voltage, and a contact portion which is in contact with the portion having conductivity of the liquid containing portion contained in the electrostatic spray main body and applies the high voltage to the liquid containing portion.
<2> An electrostatic spray device, comprising: a cartridge including a liquid containing portion which contains a liquid and a spray unit which sprays the liquid; and an electrostatic spray main body capable of containing the liquid containing portion of the cartridge, the electrostatic spray device spraying the liquid by the electrostatic spray main body applying a voltage to the liquid, the liquid containing portion of the cartridge having conductivity in at least one portion, and the electrostatic spray main body including a high-voltage generating unit which generates a high voltage, and a contact portion which is in contact with the portion having conductivity of the liquid containing portion contained in the electrostatic spray main body and applies the high voltage to the liquid containing portion.
<3> The electrostatic spray device according to <1> or <2>, in which the spray unit includes a flow path circulating the liquid which flows from the liquid containing portion, and a flow path forming member forming the flow path contains an insulating resin.
<4> The electrostatic spray device according to any one of <1> to <3>, in which the electrostatic spray main body includes a biasing portion biasing the contact portion against the liquid containing portion contained in the electrostatic spray main body.
<5> The electrostatic spray device according to <4>, in which the liquid containing portion includes a flexible region which is deformable in accordance with a reduction in an amount of a liquid composition that is contained, and the biasing portion is configured to bias the contact portion against the flexible region.
<6> The electrostatic spray device according to any one of <1> to <5>, in which the liquid containing portion is a pouch-shaped liquid containing pouch formed by stacking a plurality of sheet bodies and by joining circumferential edge portions.
<7> The electrostatic spray device according to <6>, in which each of the sheet bodies includes a laminated body including an inner layer which forms an inner surface at the time of being in the shape of a pouch, an outer layer which forms an outer surface, and a conductive layer which is disposed between the inner layer and the outer layer.
<8> The electrostatic spray device according to <7>, in which the inner layer is a sealant layer.
<9> The electrostatic spray device according to <7> or <8>, in which the conductive layer is an aluminum sheet or a vapor-deposited film in which a material selected from aluminum, aluminum oxide, conductive silica, indium tin oxide (ITO), zinc oxide (ZnO), or tin dioxide (SnO₂) is vapor-deposited on a resin film.
<10> The electrostatic spray device according to any one of <7> to <9>, in which the conductive layer has volume resistivity of less than or equal to 10⁻² Ωm.
<11> The electrostatic spray device according to any one of <7> to <10>, in which the conductive layer preferably occupies a range of greater than or equal to 5%, more preferably occupies a range of greater than or equal to 10%, even more preferably occupies a range of greater than or equal to 30%, and is still even more preferably provided over an entire surface, with respect to the entire liquid containing portion.
<12> The electrostatic spray device according to any one of <1> to <11>, in which the liquid containing portion includes a circumferential edge portion which is not deformed in accordance with a volume change in a content, and a flexible region which is deformable in accordance with the volume change in the content.
<13> The electrostatic spray device according to any one of <1> to <12>, in which in the electrostatic spray main body, a field electrode covering the entire liquid containing portion in which the liquid composition is contained is not disposed.
<14> The electrostatic spray device according to any one of <1> to <13>, in which the contact portion is disposed in a position in contact with the circumferential edge portion which is not deformed in accordance with the volume change in the content of the liquid containing portion.
<15> The electrostatic spray device according to any one of <1> to <14>, in which a contact area of the contact portion with respect to the liquid containing portion is greater than or equal to 2 mm².
<16> The electrostatic spray device according to any one of <1> to <15>,
   in which the electrostatic spray main body includes a housing including a containing space which is capable of containing the liquid containing portion of the cartridge and an insertion hole which allows the liquid containing portion to be inserted and removed with respect to the containing space, and a cover to be mounted on a surface of the housing on a side on which the insertion hole is formed, the cover includes an abutting edge portion along a circumferential edge of the housing and is configured to allow the spray of the liquid from the spray unit in a state of being mounted on the housing, and the housing includes an insulating antistatic wall erected between the insertion hole and the circumferential edge.
<17> The electrostatic spray device according to <16>,
   in which a height of the antistatic wall is higher than that of a position of a shoulder portion that is a portion of the liquid containing portion on a side close to the spray unit, in a state in which the liquid containing portion is contained in the containing space and the cartridge is mounted on the housing.
<18> The electrostatic spray device according to <16> or <17>,
   in which the height of the antistatic wall is a height at which the antistatic wall is not in contact with the cover mounted on the housing.
<19> The electrostatic spray device according to any one of <16> to <18>,
   in which the antistatic wall extends along the circumferential edge of the housing.
<20> The electrostatic spray device according to any one of <16> to <19>,
   in which a groove extending along a direction that is along the circumferential edge of the housing is formed in the antistatic wall.

### Reference Signs List

- 10: Electrostatic spray device
- 100: Cartridge
- 110: Liquid containing pouch (liquid containing portion)
- 120: Spray unit
- 121: Spray body
- 121a, 121b: Member
- 122: Connection body
- 123: Nozzle
- 123a: Spray hole
- 131: Flow path forming plate (flow path forming member)
- 132: Flow path groove
- 133: Gear pump
- 134: Sealing plate (flow path forming member)
- 200: Electrostatic spray main body
- 210: Housing
- 220: Containing space
- 223: Insertion hole
- 243: Power source unit
- 244: High-voltage generating unit
- 245: Contact portion
- 246: Biasing portion
- 247: Driving unit

## Claims

1. An electrostatic spray device, comprising:
an electrostatic spray main body capable of containing a liquid containing portion of a cartridge, the cartridge including the liquid containing portion which contains a liquid and a spray unit which sprays the liquid, the electrostatic spray device spraying the liquid by the electrostatic spray main body applying a voltage to the liquid,
the cartridge having conductivity in at least one portion of the liquid containing portion, and
the electrostatic spray main body including a high-voltage generating unit which generates a high voltage, and a contact portion which is in contact with the portion having conductivity of the liquid containing portion contained in the electrostatic spray main body and applies the high voltage to the liquid in the liquid containing portion through the portion having conductivity.

2. An electrostatic spray device, comprising: a cartridge including a liquid containing portion which contains a liquid and a spray unit which sprays the liquid; and an electrostatic spray main body capable of containing the liquid containing portion of the cartridge, the electrostatic spray device spraying the liquid by the electrostatic spray main body applying a voltage to the liquid,
the liquid containing portion of the cartridge having conductivity in at least one portion, and
the electrostatic spray main body including a high-voltage generating unit which generates a high voltage, and a contact portion which is in contact with the portion having conductivity of the liquid containing portion contained in the electrostatic spray main body and applies the high voltage to the liquid in the liquid containing portion through the portion having conductivity.

3. The electrostatic spray device according to claim 2,
wherein the spray unit includes a flow path circulating the liquid which flows from the liquid containing portion, and a flow path forming member forming the flow path contains an insulating resin.

4. The electrostatic spray device according to any one of claims 1 to 3,
wherein the electrostatic spray main body includes a biasing portion biasing the contact portion against the liquid containing portion contained in the electrostatic spray main body.

5. The electrostatic spray device according to claim 4,
wherein the liquid containing portion includes a flexible region which is deformable in accordance with a reduction in an amount of a liquid composition that is contained, and
the biasing portion is configured to bias the contact portion against the flexible region.

6. The electrostatic spray device according to any one of claims 1 to 5,
wherein the liquid containing portion is a pouch-shaped liquid containing pouch formed by stacking a plurality of sheet bodies and by joining circumferential edge portions.

7. The electrostatic spray device according to claim 6,
wherein each of the sheet bodies includes a laminated body including an inner layer which forms an inner surface at the time of being in the shape of a pouch, an outer layer which forms an outer surface, and a conductive layer which is disposed between the inner layer and the outer layer.

8. The electrostatic spray device according to claim 7,
wherein the conductive layer preferably occupies a range of greater than or equal to 5%, more preferably occupies a range of greater than or equal to 10%, even more preferably occupies a range of greater than or equal to 30%, and is still even more preferably provided over an entire surface, with respect to the entire liquid containing portion.

9. The electrostatic spray device according to any one of claims 1 to 8,
wherein the liquid containing portion includes a circumferential edge portion which is not deformed in accordance with a volume change in a content, and a flexible region which is deformable in accordance with the volume change in the content.

10. The electrostatic spray device according to any one of claims 1 to 9,
wherein in the electrostatic spray main body, a field electrode covering the entire liquid containing portion in which the liquid composition is contained is not disposed.

11. The electrostatic spray device according to any one of claims 1 to 10,
wherein the contact portion is disposed in a position in contact with the circumferential edge portion which is not deformed in accordance with the volume change in the content of the liquid containing portion.

12. The electrostatic spray device according to any one of claims 1 to 11,
wherein the electrostatic spray main body includes a housing including a containing space which is capable of containing the liquid containing portion of the cartridge and an insertion hole which allows the liquid containing portion to be inserted and removed with respect to the containing space, and a cover to be mounted on a surface of the housing on a side on which the insertion hole is formed,
the cover includes an abutting edge portion along a circumferential edge of the housing and is configured to allow the spray of the liquid from the spray unit in a state of being mounted on the housing, and
the housing includes an insulating antistatic wall erected between the insertion hole and the circumferential edge.
